(19) **Europäisches Patentamt / European Patent Office / Office européen des brevets**

(11) **EP 1 824 504 B1**

(12) # FASCICULE DE BREVET EUROPEEN

(45) Date de publication et mention de la délivrance du brevet:
**25.03.2009 Bulletin 2009/13**

(21) Numéro de dépôt: **05824581.2**

(22) Date de dépôt: **02.12.2005**

(51) Int Cl.:
**A61K 38/17** *(2006.01)*    **A61K 31/505** *(2006.01)*
**A61P 35/00** *(2006.01)*

(86) Numéro de dépôt international:
**PCT/FR2005/003005**

(87) Numéro de publication internationale:
**WO 2006/059012 (08.06.2006 Gazette 2006/23)**

(54) **COMBINAISONS ANTITUMORALES CONTENANT VEGF-TRAP ET DU 5FU OU UN DE SES DERIVES**

ANTITUMOR-KOMBINATIONEN MIT VEGF-TRAP UND 5FU ODER EINEM SEINER DERIVATE

ANTITUMOR COMBINATIONS CONTAINING VEGF-TRAP AND 5FU OR ONE OF ITS DERIVATIVES

(84) Etats contractants désignés:
**AT BE BG CH CY CZ DE DK EE ES FI FR GB GR HU IE IS IT LI LT LU LV MC NL PL PT RO SE SI SK TR**
Etats d'extension désignés:
**AL BA HR MK YU**

(30) Priorité: **03.12.2004 FR 0412870**

(43) Date de publication de la demande:
**29.08.2007 Bulletin 2007/35**

(73) Titulaire: **Aventis Pharma S.A.**
**92160 Antony (FR)**

(72) Inventeurs:
- **VRIGNAUD, Patricia**
  **F-77380 Combs La Ville (FR)**
- **CHIRON-BLONDEL, Marielle**
  **F-75015 Paris (FR)**
- **BISSERY, Marie-christine**
  **F-94400 Vitry Sur Seine (FR)**
- **FURFINE, Eric**
  **Croton On Hudson, New York 10520 (US)**
- **HOLASH, Jocelyn**
  **Alameda, California 94502 (US)**
- **CEDARBAUM, Jesse M.**
  **Larchmont, New York 10538 (US)**

(74) Mandataire: **Le Pennec, Magali et al**
**Aventis Pharma S.A.**
**Direction des Brevets**
**Tri LEO/144**
**20 Avenue Raymond Aron**
**92165 Antony Cedex (FR)**

(56) Documents cités:
**WO-A-20/05000895**    **US-A1- 2004 014 667**
**US-A1- 2005 032 699**

- **HURWITZ H ET AL: "BEVACIZUMAB PLUS IRINOTECAN, FLUOROURACIL, AND LEUCOVORIN FOR METASTATIC COLORECTAL CANCER" NEW ENGLAND JOURNAL OF MEDICINE, MASSACHUSETTS MEDICAL SOCIETY, BOSTON, MA, US, vol. 350, no. 23, 3 juin 2004 (2004-06-03), pages 2335-2342, XP009038752 ISSN: 1533-4406 cité dans la demande**
- **KABBINAVAR F ET AL: "Phase II, randomized trial comparing bevacizumab plus fluorouracil (FU)/leucovorin (LV) with FU/LV alone in patients with metastatic colorectal cancer" JOURNAL OF CLINICAL ONCOLOGY, GRUNE AND STRATTON, NEW YORK, NY, US, vol. 21, no. 1, 1 janvier 2003 (2003-01-01), pages 60-65, XP002302378 ISSN: 0732-183X**
- **BRAUN A H ET AL: "NEW SYSTEMIC FRONTLINE TREATMENT FOR METASTATIC COLORECTAL CARCINOMA" CANCER, AMERICAN CANCER SOCIETY, PHILADELPHIA, PA, US, vol. 100, no. 8, 15 avril 2004 (2004-04-15), pages 1558-1577, XP009038748 ISSN: 0008-543X**
- **KONNER JASON ET AL: "Use of soluble recombinant decoy receptor vascular endothelial growth factor trap (VEGF Trap) to inhibit vascular endothelial growth factor activity." CLINICAL COLORECTAL CANCER. OCT 2004, vol. 4 Suppl 2, octobre 2004 (2004-10), pages S81-S85, XP009050556 ISSN: 1533-0028**

EP 1 824 504 B1

- ANON.: "Capecitabine / bevacizumab compared to capecitabine alone in pretreated metastatic breast cancer : Results of a phase III study" CLINICAL BREAST CANCER , 3(6), 375-377 CODEN: CBCLB7; ISSN: 1526-8209, 2003, XP009050577
- KAKLAMANI, VIRGINIA G. ET AL: "Role of capecitabine (Xeloda) in breast cancer" EXPERT REVIEW OF ANTICANCER THERAPY , 3(2), 137-144 CODEN: ERATBJ; ISSN: 1473-7140, 2003, XP009050576
- SEMELA D ET AL: "Angiogenesis and hepatocellular carcinoma" JOURNAL OF HEPATOLOGY, MUNKSGAARD INTERNATIONAL PUBLISHERS, COPENHAGEN, DK, vol. 41, no. 5, novembre 2004 (2004-11), pages 864-880, XP004617769 ISSN: 0168-8278
- FERON O: "Targeting the tumor vascular compartment to improve conventional cancer therapy" TRENDS IN PHARMACOLOGICAL SCIENCES, ELSEVIER, AMSTERDAM, NL, vol. 25, no. 10, octobre 2004 (2004-10), pages 536-542, XP004570838 ISSN: 0165-6147

**Description**

La présente invention concerne les combinaisons de VEGF Trap et un agent chimiotoxique de classe du 5 fluorouracile ou des 5 fluoropyrimidines utiles dans le traitement des maladies néoplastiques.

Les inhibiteurs de VEGF qui sont des inhibiteurs du facteur de croissance vasculaire endothélial sont dans la majorité des cas des produits biologiques choisis parmi les récepteurs solubles, les antisens, les aptameères de RNA et les anticorps. Les dérivés des 5 fluoropyrimidines sont choisis parmi le 5-fluorouracile, la capecitabine ou la gemcitabine qui présentent des propriétés antitumorales et antileucémiques remarquables, ils sont particulièrement utiles dans le traitement des cancers de l'ovaire, du sein, du poumon ou du colon. La présente combinaison vise en particulier le traitement du cancer du colon ou de l'estomac.

La description et la préparation de l'inhibiteur du VEGF utilisé dans l'invention qui est une protéine chimère VEGF-Trap est décrite dans la demande de brevet WO00/75319. Il y a plusieurs formes de réalisation de la protéine chimère. La forme de réalisation correspondant au VEGF-Trap est celle décrite à la figure 24 (séquence). Le VEGF Trap utilisé dans l'invention est une protéine de fusion comprenant la séquence signal du VEGFR1 fusionnée au domaine Ig D2 du récepteur VEGF1, elle-même fusionnée au domaine Ig D3 du récepteur VEGFR2 fusionnée à son tour au domaine Fc de IgG1 encore appelée VEGFR1R2-Fc$\Delta$C1 ou le F1t1D2.F1k1D3.Fc$\Delta$C1.

Généralement les doses utilisées, qui dépendent des facteurs propres au sujet à traiter, sont comprises entre 20 et 800 microgrammes par kilo quand l'administration est faite par voie sous cutanée et de 2 à 20 microgrammes par kilo quand l'administration est faite par voie intraveineuse ou éventuellement par voie intranasale à une dose inférieure de l'ordre de 0,01 picogramme à 1 mg par kilo.

Le 5 fluorouracile est généralement utilisé par voie intraveineuse à une dose comprise entre 500 mg/m$^2$ et 5000 mg/m$^2$ par semaine en ce qui concerne les dérivés de 5-fluoropyrimidines tels que la capecitabine ils sont utillisés pour cette dernière généralement par voie orale à une dose comprise entre 500 et 3000 mg/m$^2$ administrés généralement en deux doses journalières. La gemcitabine est utilisée généralement par voie intraveineuse à une dose comprise entre 500 et 2000 mg/m$^2$ par semaine.

Il a été décrit dans un article de H Hurwitz, L Fehrenbacher, W Novotny, T Cartwright, J Hainsworth, W Heim, J Berlin, A Baron, S Griffing, E Holmgren, N Ferrara, G Fyfe, B Rogers, R Ross, F Kabbinavar paru dans « The New England Journal of Médecine » un essai clinique prouvant un meilleur taux de survie lors de l'utilisation de la combinaison de bevacizumab avec irinotecan, 5FU et leucoveurin par rapport à la même combinaison ne contenant pas le bevacizumab. Rien ne prouve dans cet essais clinique que l'amélioration du taux de survie provient de la combinaison de 5FU avec le bevacizumab, elle peut aussi bien provenir de la combinaison de l'irinotecan ou du leucovorin avec le bevacizumab ou peut provenir de la quatruple combinaison. Or comme il est connu que chacun des agents anticancéreux apporte à côté de son effet thérapeutique des effets secondaires toxiques il semble opportun de limiter au maximum leur présence surtout quand le même effet peut être obtenu en l'absence d'au moins l'un d'entre eux. Dans le cas présent il est connu que l'irinotecan entraîne des diarrhées importantes qui ont parfois imposé l'arrêt du traitement. D'autre part cet article ne prouve aucun effet synergique au sens de Corbett c'est à dire un effet qui ne peut pas être obtenu avec chacun des éléments de la combinaison utilisé seul à sa dose maximale tolérée.

Il a maintenant été trouvé, et c'est ce qui fait l'objet de la présente invention, que l'efficacité de VEGF TRAP peut être considérablement améliorée lorsqu'administré en association avec au moins un dérivé des 5-fluoropyrimidines (substance thérapeutiquement utile dans les traitements anticancéreux ayant un mécanisme d'action différent de celui des inhiteurs du VEGF).

Par ailleurs, l'activité des produits dépendant des doses utilisées, il est possible d'utiliser des doses plus élevées et d'augmenter l'activité en diminuant les phénomènes de toxicité ou en retardant leur apparition en associant au VEGF TRAP, d'autres substances thérapeutiquement actives des facteurs de croissance de type hématopoïétique tels que le G-CSF ou le GM-CSF ou certaines interleukines.

Plus particulièrement, l'invention concerne les associations de VEGF Trap avec le 5 flurouracile ou ses dérivés tels que la capécitabine ou la gemcitabine. Elle concerne également les combinaisons incluant en plus l'acide folinique généralement associé avec le 5-FU.

L'efficacité améliorée d'une combinaison selon l'invention peut être mise en évidence par la détermination du synergisme thérapeutique.

Une combinaison manifeste un synergisme thérapeutique s'il est thérapeutiquement supérieur à l'un ou l'autre des constituants utilisé à sa dose optimale [T.H. CORBETT et coll., Cancer Treatment Reports, 66, 1187 (1982)].

Pour mettre en évidence l'efficacité d'une combinaison, il peut être nécessaire de comparer la dose maximale tolérée de la combinaison à la dose maximale tolérée de chacun des constituants isolés dans l'étude considérée. Cette efficacité peut être quantifiée, par exemple par le $\log_{10}$ des cellules tuées qui est déterminé selon la formule suivante :

$$\log_{10} \text{ des cellules tuées} = \text{T-C (jours)}/3.32 \text{ X } T_d$$

dans laquelle T - C représente le délai de croissance des cellules qui est le temps moyen, en jours, pour que les tumeurs du groupe traité (T) et les tumeurs du groupe témoin (C) aient atteint une valeur prédéterminée (1 g par exemple) et $T_d$ représente le temps, en jours, nécessaire au doublement du volume de la tumeur chez les animaux témoins [T.H. CORBETT et coll., Cancer, 40, 2660.2680 (1977) ; F.M. SCHABEL et coll., Cancer Drug Development, Part B, Methods in Cancer Research, 17, 3-51, New-York, Academic Press Inc. (1979)] Un produit est considéré comme actif si $\log_{10}$ des cellules tuées est supérieur ou égal à 0,7. Un produit est considéré comme très actif si le $\log_{10}$ des cellules tuées est supérieur à 2,8.

La combinaison, utilisée à sa dose maximale tolérée propre, dans laquelle chacun des constituants sera présent à une dose généralement inférieure ou égale à sa dose maximale tolérée, manifestera une synergie thérapeutique lorsque le $\log_{10}$ des cellules tuées sera supérieur à la valeur du $\log_{10}$ des cellules tuées du meilleur constituant lorsqu'il est administré seul.

L'efficacité des combinaisons sur les tumeurs solides peut être déterminée expérimentalement de la manière suivante :

Les animaux soumis à l'expérience, généralement des souris, sont greffés bilatéralement par voie sous-cutanée avec 30 à 60 mg d'un fragment de tumeur mammaire MC 13/C au jour 0. Les animaux portant les tumeurs sont randomisés avant d'être soumis aux divers traitements et contrôles. Dans le cas de traitement de tumeurs avancées, on laisse les tumeurs se développer jusqu'à la taille désirée, les animaux ayant des tumeurs insuffisamment développées étant éliminés. Les animaux sélectionnés sont répartis au hasard pour subir les traitements et les contrôles. Des animaux non porteurs de tumeurs peuvent être également soumis aux mêmes traitements que les animaux porteurs afin de pouvoir dissocier l'effet toxique de l'effet propre sur la tumeur. La chimiothérapie commence généralement de 3 à 22 jours après la greffe tumorale selon le type de tumeur et les animaux sont observés tous les jours. Les différents groupes d'animaux sont pesés trois ou quatre fois par semaine jusqu'à ce que la perte maximale de poids soit atteinte puis les groupes sont pesés au moins une fois par semaine jusqu'à la fin de l'essai.

Les tumeurs sont mesurées deux ou trois fois par semaine jusqu'à ce que la tumeur atteigne environ 2 g ou jusqu'à la mort de l'animal si celle-ci survient avant que la tumeur atteigne 2 g. Les animaux sont autopsiés lors du sacrifice.

L'activité antitumorale est déterminée en fonction des différents paramètres enregistrés.

Pour l'étude des combinaisons sur des leucémies, les animaux sont greffés avec un nombre déterminé de cellules et l'activité antitumorale est déterminée par l'augmentation du temps de survie des souris traitées par rapport aux témoins. Un produit est considéré comme actif si le temps d'augmentation de survie est supérieur à 27 % et il est considéré comme très actif s'il est supérieur à 75 % dans le cas de la leucémie P388.

A titre d'exemples, sont donnés, dans les tableaux suivants les résultats obtenus avec des combinaisons du VEGF Trap et du 5-fluorouracile utilisées à leur dose optimale.

La présente invention concerne également les compositions pharmaceutiques contenant les combinaisons selon l'invention.

Les produits qui constituent la combinaison peuvent être administrés simultanément, séparément ou d'une manière étalée dans le temps de façon à obtenir le maximum d'efficacité de la combinaison ; chaque administration pouvant avoir une durée variable allant d'une administration totale rapide à une perfusion continue.

Il en résulte que, au sens de la présente invention, les combinaisons ne sont pas uniquement limitées à celles qui sont obtenues par association physique des constituants, mais aussi à celles qui permettent une administration séparée qui peut être simultanée ou étalée dans le temps.

Les compositions selon l'invention sont de préférence les compositions administrables par voie parentérale. Cependant, ces compositions peuvent être administrées par voie orale.

Les compositions pour administration parentérale sont généralement des solutions ou des suspensions stériles pharmaceutiquement acceptables qui peuvent éventuellement être préparées extemporanément au moment de l'emploi. Pour la préparation de solutions ou de suspensions non aqueuses peuvent être utilisées des huiles végétales naturelles telles que l'huile d'olive, l'huile de sésame ou l'huile de paraffine ou les esters organiques injectables tels que l'oléate d'éthyle. Les solutions stériles aqueuses peuvent être constituées d'une solution du produit dans l'eau. Les solutions aqueuses conviennent pour l'administration intraveineuse dans la mesure où le pH est convenablement ajusté et où l'isotonicité est réalisée, par exemple par une quantité suffisante de chlorure de sodium ou de glucose. La stérilisation peut être réalisée par chauffage ou par tout autre moyen qui n'altère pas la composition. Les combinaisons peuvent aussi se présenter sous forme de liposomes ou sous forme d'association avec des supports tels que les cyclodextrines ou les polyéthylèneglycols.

Dans les combinaisons selon l'invention dont l'application des constituants peut être simultanée, séparée ou étalée dans le temps, il est particulièrement avantageux que la quantité de dérivé du VEGF Trap représente de 2 à 80% en poids de la combinaison cette teneur pouvant varier en fonction de la nature de la substance associée, de l'efficacité recherchée et de la nature du cancer à traiter.

Les combinaisons selon l'invention sont particulièrement utiles dans le traitement des cancers du colon et/ou de l'estomac. En particulier, elles peuvent présenter l'avantage de pouvoir mettre en oeuvre les constituants à des doses nettement plus faibles que celles auxquelles ils sont utilisés seuls.

L'exemple suivant illustre une combinaison selon l'invention.

EXEMPLE

On prépare selon la technique habituelle, pour l'administration sous cutanée, des ampoules de 1 cm3 contenant 25 mg de VEGF Trap qui sont diluées dans un tampon phosphate.

On prépare selon la technique habituelle, pour l'administration intraveineuse 0,2ml par souris à partir d'une solution commerciale de 5 cm3 contenant 250 mg de 5 FU à diluer avec du glucose 5 % dans l'eau.

Ces solutions sont administrées simultanément, après dilution convenable, par perfusion.

Le traitement peut être répété plusieurs fois par jour ou par semaine jusqu'à une rémission partielle ou totale ou une guérison.

| Dosage en mg/kg/jour (total dose in mg/kg) | | Mort due au traitement | % perte poids au nadir | T/C % | T-C jours | lck |
|---|---|---|---|---|---|---|
| sc VEGF-trap (jour 4, 7, 11, 14, 18, 21) | iv 5-FU (jour 4, 11, 18) | | | | | |
| 40 (240) | - | 0/5 | 2.3 | 4 | 12.7 | 1.4 |
| 25 (150) | - | 0/5 | 1.1 | 8 | 13.9 | 1.5 |
| 10 (60) | - | 0/5 | 1.1 | 9 | 12.1 | 1.3 |
| 2.5 (15) | - | 0/5 | 0.9 | 32 | 5.2 | 0.6 |
| - | 145 (435) | 1/5 | 8.7 | Toxic | - | - |
| - | 90 (270) | 0/5 | 4.8 | 0 | 12.5 | 1.3 |
| - | 55.8 (167.4) | 0/5 | 1.1 | 12 | 7.8 | 0.8 |
| - | 34.6 (103.8) | 0/5 | + 2.3 | 34 | 4.6 | 0.5 |
| 40 (240) | 90 (270) | 0/5 | 8.0 | 0 | 25.2 | 2.7 |
| 25 (150) | 90 (270) | 0/5 | 9.6 | 0 | 24.8 | 2.7 |
| 10 (60) | 90 (270) | 0/5 | 7.4 | 0 | 23.1 | 2.5 |
| 10 (60) | 55.8 (167.4) | 0/5 | 4.4 | 0 | 16.5 | 1.8 |
| 10 (60) | 34.6 (103.8) | 0/5 | 5.4 | 1 | 20.0 | 2.2 |
| 2.5 (15) | 90 (270) | 0/5 | 7.0 | 0 | 20.4 | 2.2 |
| 2.5 (15) | 55.8 (167.4) | 0/5 | 2.0 | 0 | 18.0 | 1.9 |
| 2.5 (15) | 34.6 (103.8) | 0/5 | 2.0 | 6 | 11.7 | 1.3 |

BCM-1428 (05/28/04 - 07.30.2004): Temps de doublement de la tumeur = 2.8 jours.
Temps moyen pour 750 mg sur controles = 22.2 j. Durée de traitement = 18 j pour VEGF-trap et combination, et 15 days pour 5-FU.
Abbreviations utilisées: T/C = inhibition de la croissance de la tumeur au jour 24, (T-C) délai de croissance de la tumeur, lck = log cell tuées.

**Revendications**

1. Combinaisons présentant un effet synergique contenant le VEGF Trap avec au moins du 5-fluorouracile ou un dérivé de 5 fluoropyrimidine pour leur utilisation dans le traitement des maladies néoplastiques.

2. Combinaisons présentant un effet synergique contenant le VEGF Trap avec le 5-fluorouracile ou la capecitabine ou la gemcitabine.

3. Combinaisons présentant un effet synergique selon la revendication 2 contenant le VEGF Trap avec le fluorouracile.

**4.** Combinaisons présentant un effet synergique pour leur utilisation selon la revendication 1 ou combinaisons présentant un effet synergique selon l'une des revendications 2 et 3 **caractérisées en ce qu'**elles contiennent en outre de l'acide folinique.

**5.** Combinaisons présentant un effet synergique pour leur utilisation selon l'une des revendications 1 et 4 ou combinaisons présentant un effet synergique selon l'une des revendications 2, 3 et 4 **caractérisées en ce qu'**elles contiennent de 2 à 80 % en poids de VEGF Trap.

**6.** Combinaisons pour leur utilisation selon les revendications 1, 4 et 5 ou combinaisons selon les revendications 2, 3, 4 et 5 contenant le VEGF Trap avec du 5-flurorouracile ou un dérivé de 5 fluoropyrimidine et de l'acide folinique à l'exclusion de tout autre dérivé chimiotoxique présentant un effet thérapeutiquement synergique dans le traitement des maladies néoplasiques.

**7.** Produits contenant du VEGF Trap et au moins une substance thérapeutiquement utile telle que définie dans l'une des revendications 1 à 4 dans le traitement des maladies néoplasiques comme préparation combinée pour une utilisation simultanée, séparée ou étalée dans le temps en thérapie anticancéreuse.

**Claims**

**1.** Combinations exhibiting a synergistic effect containing VEGF-Trap with at least 5-fluorouracil or a 5-fluoropyrimidine derivative, for their use in the treatment of neoplastic diseases.

**2.** Combinations exhibiting a synergistic effect containing VEGF-Trap with 5-fluorouracil or capecitabine or gemcitabine.

**3.** Combinations exhibiting a synergistic effect according to Claim 2, containing VEGF-Trap with fluorouracil.

**4.** Combinations exhibiting a synergistic effect for their use according to Claim 1 or combinations exhibiting a synergistic effect according to either of Claims 2 and 3, **characterized in that** they also contain folinic acid.

**5.** Combinations exhibiting a synergistic effect for their use according to either of Claims 1 and 4 or combinations exhibiting a synergistic effect according to one of Claims 2, 3 and 4, **characterized in that** they contain from 2% to 80% by weight of VEGF-Trap.

**6.** Combinations for their use according to Claims 1, 4 and 5 or combinations according to Claims 2, 3, 4 and 5 containing VEGF-Trap with 5-flurorouracil or a 5-fluoropyrimidine derivative and folinic acid with the exclusion of any other chemotoxic derivative exhibiting a therapeutically synergistic effect in the treatment of neoplastic diseases.

**7.** Products containing VEGF-Trap and at least one therapeutically useful substance as defined in one of Claims 1 to 4, in the treatment of neoplastic diseases, as a combined preparation for simultaneous use, separate use or use spread out over time in anticancer therapy.

**Patentansprüche**

**1.** Kombinationen mit synergistischer Wirkung, enthaltend VEGF Trap zusammen mit mindestens 5-Fluoruracil oder einem 5-Fluorpyrimidinderivat, zur Verwendung bei der Behandlung von neoplastischen Krankheiten.

**2.** Kombinationen mit synergistischer Wirkung, enthaltend VEGF Trap gemeinsam mit 5-Fluoruracil oder Capecitabin oder Gemcitabin.

**3.** Kombinationen mit synergistischer Wirkung nach Anspruch 2, enthaltend VEGF Trap gemeinsam mit Fluoruracil.

**4.** Kombinationen mit synergistischer Wirkung zur Verwendung nach Anspruch 1 oder Kombinationen mit synergistischer Wirkung nach einem der Ansprüche 2 und 3, **dadurch gekennzeichnet, daß** sie weiterhin Folinsäure enthalten.

**5.** Kombinationen mit synergistischer Wirkung zur Verwendung nach einem der Ansprüche 1 und 4 oder Kombinationen mit synergistischer Wirkung nach einem der Ansprüche 2, 3 und 4, **dadurch gekennzeichnet, daß** sie 2 bis 80 Gew.-% VEGF Trap enthalten.

6. Kombinationen zur Verwendung nach den Ansprüchen 1, 4 und 5 oder Kombinationen nach den Ansprüchen 2, 3, 4 und 5, enthaltend VEGF Trap gemeinsam mit 5-Fluoruracil oder einem 5-Fluorpyrimidinderivat sowie Folinsäure unter Ausschluß jeglichen sonstigen chemotoxischen Derivats mit therapeutisch synergistischer Wirkung bei der Behandlung von neoplastischen Krankheiten.

7. Produkte enthaltend VEGF Trap sowie mindestens eine therapeutisch nützliche Substanz wie in einem der Ansprüche 1 bis 4 definiert bei der Behandlung von neoplastischen Krankheiten als Kombinationspräparat für die gleichzeitige, getrennte oder zeitlich gestaffelte Verwendung bei der Antikrebstherapie.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 0075319 A

**Littérature non-brevet citée dans la description**

- **T.H. CORBETT.** *Cancer Treatment Reports,* 1982, vol. 66, 1187
- **T.H. CORBETT.** *Cancer,* 1977, vol. 40, 2660-2680
- **F.M. SCHABEL.** Cancer Drug Development, Part B, Methods in Cancer Research. Academic Press Inc, 1979, vol. 17, 3-51